Europäisches Patentamt
European Patent Office
Office européen des brevets

(19)

(11) Numéro de publication: **0 117 794**
**A1**

(12) **DEMANDE DE BREVET EUROPEEN**

(21) Numéro de dépôt: **84400233.7**

(22) Date de dépôt: **03.02.84**

(51) Int. Cl.³: **C 07 D 243/04**, C 07 D 405/12, C 07 D 401/12, C 07 D 403/12, A 61 K 31/55

(30) Priorité: **16.02.83 FR 8302475**

(43) Date de publication de la demande: **05.09.84 Bulletin 84/36**

(84) Etats contractants désignés: **AT BE CH DE FR GB IT LI LU NL SE**

(71) Demandeur: **LIPHA, LYONNAISE INDUSTRIELLE PHARMACEUTIQUE, 34, rue Saint Romain Boîte Postale 8481, F-69359 Lyon Cedex 08 (FR)**

(72) Inventeur: **Bayssat, Michel, Chemin de Beckensteiner, F-69260 Charbonnieres (FR)**
Inventeur: **Ferrand, Gérard, 62, rue des Aqueducs, F-69005 Lyon (FR)**
Inventeur: **Depin, Jean-Claude, 113, Cours Gambetta, F-69003 Lyon (FR)**

(74) Mandataire: **Bouton Neuvy, Liliane et al, L'Air liquide, Société Anonyme pour L'Etude et L'Exploitation des Procédés Georges Claude 75, Quai d'Orsay, F-75321 Paris Cedex 07 (FR)**

(54) **Amino-2-phényl-5 benzodiazépines-1,3 procédé de préparation et médicaments les contenant.**

(57) La présente invention concerne des amino-2 phényl-5 benzodiazépines-1,3 représentées par la formule

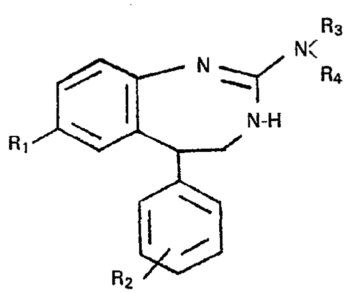

dans laquelle $R_1$ et $R_2$ sont l'hydrogène, un atome d'halogène, un radical alcoyle ou alcoxy, $R_2$ pouvant occuper l'une quelconque des positions possibles sur le noyau aromatique; $R_3$ est l'hydrogène ou un radical alcoyle et alors $R_4$ est l'hyxdrogène, un radical alcoyle, cycloalcoyle, arylalcoyle, hétéroarylalcoyle, dialcoylaminoéthyle, dialcoylaminopropyle, (éthyl-1 pyrrolidino-2) méthyle, dialcoylamino, alcoxycarbonyle ou cyano ou bien $R_3$ et $R_4$ forment avec l'atome d'azote adjacent un hétérocycle ayant de 5 à 7 chaînons, contenant éventuellement un autre hétéroatome choisi parmi l'oxygène, le soufre ou l'azote, ce dernier pouvant être substitué par un groupe alcoyle.

Application de ces composés comme médicaments antidépresseurs.

EP 0 117 794 A1

La présente invention concerne de nouvelles amino-2 phényl-5 dihydro-4,5-3H-benzodiazépines-1,3, un procédé permettant de les préparer et leur application dans le domaine thérapeutique.

H.R. RODRIGUEZ et Coll. ont décrit l'amino-2 diméthoxy-7,8 dihydro-4,5-3H-benzodiazépine-1,3 /J.Org.Chem. 33, 670, (1968)/, et dans le brevet US.3.681.340 mentionnent l'effet dépresseur du système nerveux central et coronarodilatateur de certaines benzodiazépines. Quelques autres amino-2 dihydro-4,5-3H-benzodiazépines-1,3 non substituées en position-5 ont déjà été décrites, notamment le brevet américain 3.780.023 a pour objet des 2-aralkylamino-4,5 dihydro-3H-1,3 benzodiazépines qui sont des dépresseurs du système nerveux central possédant une activité tranquillisante. Le brevet américain 3.780.024 concerne des 2-aminoalkylamino-4,5 dihydro-3H-1,3 benzodiazépines présentés comme des agents anti-hypertenseurs. Le brevet US 3.828.122 décrit des 2-hétérocycliques-1,3 benzodiazépines, dont aucune n'est substituée par un groupe phényle en position 5, et qui sont des agents antihypertenseurs.

Aucune benzodiazépine décrite dans les travaux antérieurs cités ne possède de phényle en position 5. Les méthodes de synthèse développées dans les brevets US. 3.681.340, 3.818.122, 3.818.023 et 24 nécessitent la préparation d'intermédiaires qui ne peuvent conduire aux nouveaux composés. En outre, aucun des documents connus ne fait mention d'une activité antidépressive.

Les composés de l'invention, différant de l'art antérieur par leur structure chimique et leur activité pharmacologique, sont représentés par la formule générale I :

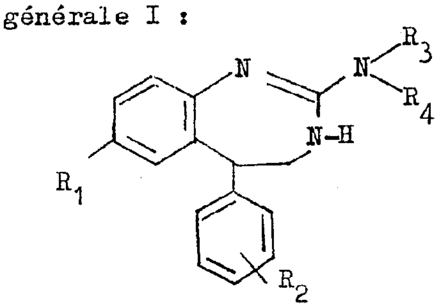

(I)

dans laquelle $R_1$ et $R_2$ sont l'hydrogène, un atome d'halogène, un radical alcoyle ou alcoxy, $R_2$ pouvant occuper l'une quelconque des positions possibles sur le noyau aromatique. $R_3$ est l'hydrogène ou un radical alcoyle et alors $R_4$ est l'hydrogène, un radical alcoyle, cycloalcoyle, arylalcoyle, hétéroarylacoyle, dialcoylaminoéthyle, dialcoylaminopropyle,(éthyl-1 pyrrolidino-2) méthyle, dialcoylamino, alcoxycarbonyle ou cyano ; ou bien $R_3$ et $R_4$ forment avec l'atome

d'azote adjacent un hétérocycle ayant de 5 à 7 chaînons, contenant éventuellement un autre hétéroatome choisi parmi l'oxygène, le soufre ou l'azote, ce dernier pouvant être substitué par un groupe alcoyle. Lorsque $R_4$ est le groupe cyano, $R_3$ ne peut être que l'hydrogène. Les termes alcoyle ou alcoxy désignent des radicaux hydrocarbonés linéaires ou ramifiés comprenant de 1 à 4 atomes de carbone. Le terme cycloalcoyle est limité à des cycles contenant de 4 à 7 atomes de carbone. Par arylalcoyle ou hétéroarylalcoyle on entend un groupe de formule II : Ar-alk , dans laquelle Ar est un noyau benzénique éventuellement mono ou polysubstitué par un halogène, un groupe alcoyle, alcoxy, nitro ou amino. Ar peut aussi être le groupe pyridyl-2, pyridyl-3, pyridyl-4 ou furyl-2; alk- représente le groupe méthylène ou une chaîne éthylène éventuellement substituée par un groupe hydroxy.

Les composés de formule I pour lesquels $R_3$ est l'hydrogène et $R_4$ est l'hydrogène ou un radical alcoxycarbonyle constituent une classe de composés particulièrement intéressante.

Les sels pharmaceutiquement acceptables font partie intégrante de l'invention. Ce peuvent être des sels préparés soit à partir d'acides minéraux comme l'acide chlorhydrique, l'acide bromhydrique, l'acide sulfurique, l'acide phosphorique, soit à partir d'acides organiques comme l'acide tartrique, l'acide citrique, l'acide acétique, l'acide maléique, l'acide fumarique, l'acide oxalique, l'acide méthane sulfonique.

Les composés de l'invention, à l'exclusion de ceux pour lesquels $R_4$ représente un groupe alcoxycarbonyle ou cyano sont obtenus par condensation des dérivés de formule générale III

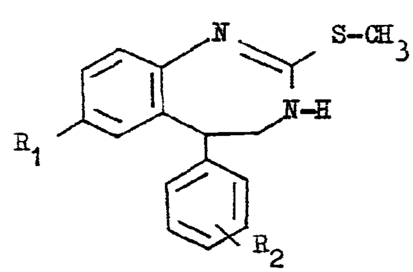

, HI (III)

avec une amine de formule générale IV 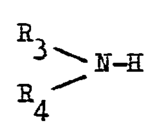 (IV)

Dans les formules III et IV, $R_1$, $R_2$, $R_3$ et $R_4$ ont les significations données précédemment. La réaction s'effectue dans un solvant inerte, à des températures comprises entre la température ambiante et la température d'ébullition du solvant utilisé. Les solvants ayant donné les meilleurs résultats sont les alcanols et les nitriles de bas poids

moléculaire et le N, N-diméthylformamide. L'utilisation de l'acétonitrile s'est révélée particulièrement avantageuse. Le temps de réaction, fonction de la température utilisée, peut varier de 1h à 60h. La préparation des composés de formule III est décrite dans la demande de brevet français 82.10.466.

Les composés de l'invention pour lesquels $R_4$ représente le groupe alcoxycarbonyle, $R_1$, $R_2$ et $R_3$ ayant les significations données précédemment s'obtiennent en condensant une amino-2 phényl-5 benzodiazépine-1,3 de formule générale V.

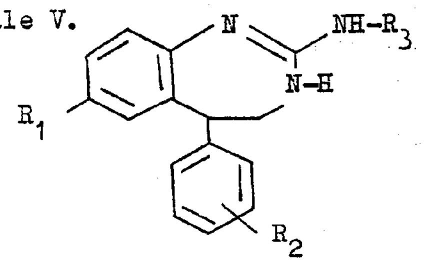

(V)

avec un chloroformiate d'alcoyle de formule VI. $Cl - COOR_5$ (VI) dans laquelle $R_5$ est un groupe alcoyle. La réaction s'effectue en présence d'une base telle que la soude diluée, la potasse diluée ou un carbonate alcalin.

Les composés de l'invention pour lesquels $R_4$ est le groupe cyano et $R_3$ est l'hydrogène (formule X) s'obtiennent selon la suite de réactions ci-dessous, dans lesquelles $R_1$ et $R_2$ ont les significations données précédemment : - une diamine de formule VII dont la préparation est décrite dans la demande de brevet précédemment citée

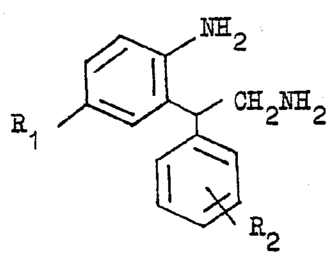

(VII)

est condensée avec le composé VIII.

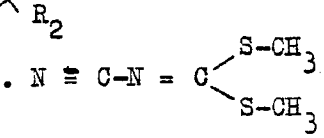

(VIII)

pour donner l'intermédiaire IX

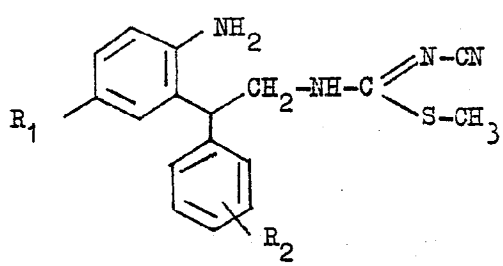

(IX)

- IX est cyclisé en X par chauffage dans un solvant de haut point d'ébullition :

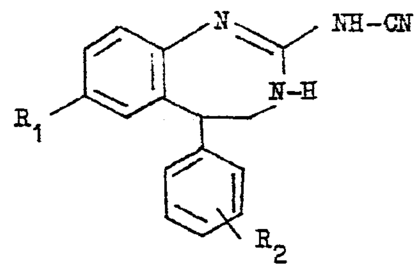

X

Les composés de l'invention représentés par la formule I exercent une action sur le système nerveux central, ce qui les rend utiles en médecine humaine dans le traitement des états dépressifs et des troubles psychiques. Cette activité modificatrice de l'humeur peut être déterminée par des tests normalisés comme l'inhibition du ptosis à la réserpine.

On provoque le ptosis chez la souris SWISS par injection I.P. de 5 mg/kg de réserpine. Ce ptosis est côté selon B. RUBIN et Coll. [J. Pharmacol. Exp. Thérap. 120, 125 (1957)] 1 h 30 plus tard. Les composés sont donnés oralement, en même temps que l'injection de réserpine. Les doses efficaces 50 (DE 50) obtenues pour quelques produits de l'invention et celles obtenues pour une substance étalon comme l'amitriptyline [chlorhydrate de (diméthylamino-3 propylidène) -5 dibenzo [a,d] cycloheptadiène-1,4] sont consignées dans le tableau I.

TABLEAU I

| Produits | Ptosis à la réserpine DE 50 (mg/kg/P.O) |
|---|---|
| AMITRIPTYLINE | 10 |
| Exemple 1 | 18 |
| Exemple 3 | 10 |
| Exemple 4 | 11 |
| Exemple 8 | 3 |
| Exemple 48 | 13 |

Les doses léthales 50 (DL 50) déterminées par voie orale sur la souris SWISS sont données dans le tableau II.

TABLEAU II

| Produits | DL 50 P.O. (mg/kg) |
|---|---|
| AMITRIPTYLINE | 150 |
| Exemple 1 | 600 |
| Exemple 3 | 330 |
| Exemple 4 | 1 200 |
| Exemple 8 | 2 400 |
| Exemple 48 | > 3 200 |

La présente demande a également pour objet l'application des composés I à titre de médicaments, et notamment de médicaments antidépresseurs. Ces médicaments peuvent être administrés par voie orale sous forme de comprimés, comprimés dragéifiés ou de gélules ou par voie rectale sous forme de suppositoires. Le principe actif est associé à divers excipients pharmaceutiquement compatibles. Les posologies journalières peuvent varier de 10 à 200 mg selon la gravité de l'affection traitée.

On donne ci-dessous, à titre d'exemple non limitatif quelques formulations pharmaceutiques :

- Composition d'un comprimé de 100 mg éventuellement enrobé :
  - . principe actif.............. 5 mg
  - . lactose..................... 41 mg
  - . amidon de blé............... 41 mg
  - . gélatine.................... 2 mg
  - . acide alginique............. 5 mg
  - . talc........................ 5 mg
  - . stéarate de magnésium....... 1 mg
- Composition d'une gélule :
  - . principe actif.............. 10 mg
  - . lactose..................... 32 mg
  - . amidon de blé............... 25 mg
  - . talc........................ 2,5 mg
  - . stéarate de magnésium....... 0,5 mg

Il est donné ci-après des exemples illustrant l'invention à titre nullement limitatif.

Exemple 1.

Amino-2 phényl-5 dihydro-4,5-3H-benzodiazépine-1,3, chlorhydrate

Un mélange de 95,6 g (241,2 m.moles) de iodhydrate de méthylthio-2 phényl-5 dihydro-4,5-3H-benzodiazépine-1,3, de 1250 cm3 d'éthanol et de 970 cm3 d'ammoniaque à 34 % est porté à reflux. Toutes les 4 heures environ un barbottage d'ammoniac est effectué jusqu'à saturation du milieu réactionnel. La réaction est poursuivie jusqu'à cessation du dégagement de méthanethiol (environ 30 heures). Après refroidissement un insoluble est filtré et le filtrat est évaporé à sec. Le résidu est repris par 500 cm3 d'eau puis alcalinisé très fortement par de la potasse en pastilles. Le mélange est agité pendant 1 heure avec 200 cm3 d'éther. Le précipité d'amino-2 phényl-5 dihydro-4,5-3H-benzodiazépine-1,3 est filtré, lavé à l'eau et séché. Il est dissous dans de

l'éthanol et traité par 50 cm3 d'acide chlorhydrique 10 N. Le mélange est évaporé à sec et le chlorhydrate d'amino-2 phényl-5 dihydro-4,5-3H-benzodiazépine-1,3 obtenu purifié par recristallisation dans un mélange acétone-éthanol. Rdt : 42,3 g (64 %), F = 215 - 217°C.

<u>Analyse centésimale</u> $C_{15}H_{16}ClN_3$ M = 273,76

| | C % | H % | Cl % | N % |
|---|---|---|---|---|
| Calculé | 65,80 | 5,89 | 12,95 | 15,35 |
| Trouvé | 65,94 | 5,98 | 12,84 | 15,18 |

Les produits décrits dans les exemples 2 à 10 sont obtenus en opérant dans les conditions de l'exemple 1.

<u>Exemple 2.</u>

<u>Amino-2 chloro-7 phényl-5 dihydro-4,5-3H-benzodiazépine-1,3, chlorhydrate</u>

Obtenu à partir de l'iodhydrate de chloro-7 méthylthio-2 phényl-5 dihydro-4,5-3H-benzodiazépine-1,3. F = 166 - 168°C (acétone-éthanol)

<u>Analyse centésimale</u> $C_{15}H_{15}Cl_2N_3$ M = 308,20

| | C % | H % | Cl % | N % |
|---|---|---|---|---|
| Calculé | 58,45 | 4,91 | 23,01 | 13,63 |
| Trouvé | 58,44 | 4,95 | 22,99 | 13,64 |

<u>Exemple 3.</u>

<u>Amino-2 (méthyl-4 phényl)-5 dihydro-4,5-3H-benzodiazépine-1,3, chlorhydrate</u>

Obtenu à partir de l'iodhydrate de (méthyl-4 phényl)-5 méthylthio-2 dihydro-4,5-3H-benzodiazépine-1,3. F = 162 - 163°C (acétone - isopropanol)

<u>Analyse centésimale</u> $C_{16}H_{18}ClN_3$ M = 287,78

| | C % | H % | Cl % | N % |
|---|---|---|---|---|
| Calculé | 66,77 | 6,30 | 12,32 | 14,60 |
| Trouvé | 66,90 | 6,40 | 12,30 | 14,61 |

<u>Exemple 4.</u>

<u>Amino-2 (chloro-4 phényl)-5 dihydro-4,5-3H-benzodiazépine-1,3, chlorhydrate</u>

Obtenu à partir de l'iodhydrate de (chloro-4 phényl)-5 méthylthio-2 dihydro-4,5-3H-benzodiazépine-1,3. F = 163 - 166°C (acétone-éthanol)

<u>Analyse centésimale</u> $C_{15}H_{15}Cl_2N_3$ M = 308,20

| | C % | H % | Cl % | N % |
|---|---|---|---|---|
| Calculé | 58,45 | 4,91 | 23,01 | 13,63 |
| Trouvé | 58,60 | 4,93 | 22,90 | 13,61 |

Exemple 5.

Amino-2 (méthoxy-4 phényl)-5 dihydro-4,5-3H-benzodiazépine-1,3, hémi-oxalate

La base est préparée en opérant selon l'exemple 1, à partir de l'iodhydrate de (méthoxy-4 phényl)-5 méthylthio-2 dihydro-4,5-3H-benzodiazépine-1,3. L'hémi-oxalate est obtenu par addition d'une solution d'un demi équivalent d'acide oxalique dans l'acétone à une solution de cette base dans l'acétone. F = 248 - 249°C (eau - éthanol).

Analyse centésimale $C_{17}H_{18}N_3O_3$ M = 312,33

| | C % | H % | N % |
|---|---|---|---|
| Calculé | 65,37 | 5,81 | 13,46 |
| Trouvé | 65,06 | 6,03 | 13,29 |

Exemple 6.

Amino-2 méthyl-7 phényl-5 dihydro-4,5-3H-benzodiazépine-1,3, chlorhydrate

Obtenu à partir de l'iodhydrate de méthyl-7 méthylthio-2 phényl-5 dihydro-4,5-3H-benzodiazépine-1,3. F = 159 - 161°C (isopropanol-éther diisopropylique).

Analyse centésimale $C_{16}H_{18}ClN_3$ M = 287,78

| | C % | H % | Cl % | N % |
|---|---|---|---|---|
| Calculé | 66,77 | 6,30 | 12,32 | 14,60 |
| Trouvé | 66,68 | 6,13 | 12,26 | 14,66 |

Exemple 7.

Amino-2 (méthyl-3 phényl)-5 dihydro-4,5-3H-benzodiazépine-1,3, chlorhydrate

Obtenu à partir de l'iodhydrate de (méthyl-3 phényl)-5 méthylthio-2 dihydro-4,5-3H-benzodiazépine-1,3. F = 137 - 139°C (acétone - éthanol).

Analyse centésimale $C_{16}H_{18}ClN_3$ M = 287,78

| | C % | H % | Cl % | N % |
|---|---|---|---|---|
| Calculé | 66,77 | 6,30 | 12,32 | 14,60 |
| Trouvé | 66,66 | 6,57 | 12,36 | 14,46 |

Exemple 8.

Amino-2 méthyl-7 (méthyl-4 phényl)-5 dihydro-4,5-3H-benzodiazépine-1,3 chlorhydrate

Obtenu à partir de l'iodhydrate de méthyl-7 (méthyl-4 phényl)-5 méthylthio-2 dihydro-4,5-3H-benzodiazépine-1,3. F = 202 - 203°C (isopropanol-éther diisopropylique).

Analyse centésimale $C_{17}H_{20}ClN_3$ M = 301,81

| | C % | H % | Cl % | N % |
|---|---|---|---|---|
| Calculé | 67,66 | 6,68 | 11,75 | 13,93 |
| Trouvé | 67,65 | 6,55 | 11,74 | 13,99 |

Exemple 9.

Amino-2 chloro-7 (méthyl-4 phényl)-5 dihydro-4,5-3H-benzodiazépine-1,3 chlorhydrate

Obtenu à partir de l'iodhydrate de chloro-7 (méthyl-4 phényl)-5 méthylthio-2 dihydro-4,5-3H-benzodiazépine-1,3. F = 172-174°C (isopropanol-éther diisopropylique).

Analyse centésimale $C_{16}H_{17}Cl_2N_3$ M = 322,23.

| | C % | H % | Cl % | N % |
|---|---|---|---|---|
| Calculé | 59,64 | 5,32 | 22,00 | 13,04 |
| Trouvé | 59,96 | 5,10 | 21,97 | 13,09 |

Exemple 10.

Amino-2 (méthyl-2 phényl)-5 dihydro-4,5-3H-benzodiazépine-1,3, chlorhydrate

Obtenu à partir de l'iodhydrate de (méthyl-2 phényl)-5 méthylthio-2 dihydro-4,5-3H-benzodiazépine-1,3. F = 243-244°C (isopropanol).

Analyse centésimale $C_{16}H_{18}ClN_3$ M = 287,78

| | C % | H % | Cl % | N % |
|---|---|---|---|---|
| Calculé | 66,77 | 6,30 | 12,32 | 14,60 |
| Trouvé | 66,89 | 6,36 | 12,33 | 14,43 |

Exemple 11.

Diméthylamino-2 phényl-5 dihydro-4,5-3H-benzodiazépine-1,3, oxalate

En opérant comme dans l'exemple 1 à partir de l'iodhydrate de méthylthio-2 phényl-5 dihydro-4,5-3H-benzodiazépine-1,3 et de la diméthylamine on obtient la diméthylamino-2 phényl-5 dihydro-4,5-3H-benzodiazépine-1,3. L'oxalate est obtenu par addition d'un équivalent d'acide oxalique à une solution acétonique de la base. F = 196-198°C (acétone-méthanol).

Analyse centésimale $C_{19}H_{21}N_3O_4$ M = 355,38

| | C % | H % | N % |
|---|---|---|---|
| Calculé | 64,21 | 5,93 | 11,82 |
| Trouvé | 63,91 | 6,17 | 11,79 |

Exemple 12.

Méthylamino-2 (méthyl-4 phényl)-5 dihydro-4,5-3H-benzodiazépine-1,3, chlorhydrate

Un mélange de 16,4 g d'iodhydrate de (méthyl-4 phényl)-5 méthylthio-2

dihydro-4,5-3H-benzodiazépine-1,3, de 100 cm3 d'éthanol et de 20cm3 d'une solution éthanolique à 33 % de méthylamine est porté à reflux. Toutes les trois heures on rajoute 100 cm3 de solution éthanolique de méthylamine et ce, jusqu'à cessation du dégagement de méthanethiol (environ 12 heures). Le milieu réactionnel est alors concentré à sec. Le résidu est repris par de l'eau et le mélange rendu fortement alcalin par addition de potasse en pastilles puis extrait deux fois au chlorure de méthylène. Les phases organiques rassemblées sont lavées à l'eau et séchées sur sulfate de sodium. Le chlorure de méthylène est éliminé, et au résidu dissous dans l'éthanol on ajoute 8 cm3 d'acide chlorhydrique 10 N. Après évaporation à sec, le chlorhydrate de méthylamino-2 (méthyl-4 phényl)-5 dihydro-4,5-3H-benzodiazépine-1,3 est recristallisé dans un mélange isopropanol-éther diisopropylique. F = 210-211°C. Rdt : 9,9 g (79 %).

Analyse centésimale $C_{17}H_{20}ClN_3$ M = 301,81

| | C % | H % | Cl % | N % |
|---|---|---|---|---|
| Calculé | 67,66 | 6,68 | 11,75 | 13,93 |
| Trouvé | 67,63 | 6,88 | 11,67 | 13,82 |

Exemple 13.

Méthylamino-2 phényl-5 dihydro-4,5-3H-benzodiazépine-1,3, oxalate

En opérant comme dans l'exemple 12 à partir de l'iodhydrate de méthylthio-2 phényl-5 dihydro-4,5-3H-benzodiazépine-1,3 on obtient après traitement la méthylamino-2 phényl-5 dihydro-4,5-3H-benzodiazépine-1,3 qui, après recristallisation dans un mélange hexane-acétate d'éthyle fond à F = 142-144°C.

A cette base dissoute dans l'acétone, on ajoute un équivalent d'acide oxalique dissous dans l'acétone. L'oxalate formé est filtré et recristallisé dans un mélange acétone-éthanol. F = 163-165°C.

Analyse centésimale $C_{18}H_{19}N_3O_4$ M = 341,35.

| | C % | H % | N % |
|---|---|---|---|
| Calculé | 63,33 | 5,61 | 12,31 |
| Trouvé | 63,40 | 5,55 | 12,38 |

Exemple 14.

Benzylamino-2 phényl-5 dihydro-4,5-3H-benzodiazépine-1,3, chlorhydrate

Un mélange de 8 g (20 m.moles) de iodhydrate de méthylthio-2 phényl-5 dihydro-4,5-3H-benzodiazépine-1,3, de 4,3 g (40 m.moles) de benzylamine et de 50 cm3 d'acétonitrile est porté à reflux, sous azote, jusqu'à cessation du dégagement de méthanethiol (environ 7 h). Après refroidissement le milieu réactionnel est dilué à l'éther. L'iodhydrate de benzylamino-2 phényl-5 dihydro-4,5-3H-benzodiazépine-1,3 précipite. Il est filtré et mis en suspension dans l'eau. Le mélange est alcalinisé par une solution de soude et extrait à l'éther. La phase organique est lavée à l'eau et séchée sur sulfate de sodium. Le solvant est évaporé et le résidu dissous dans de l'éthanol. A cette solution on ajoute 5 cm3 d'une solution 10 N d'acide chlorhydrique et concentre sous vide. On obtient le chlorhydrate de benzylamino-2 phényl-5 dihydro-4,5-3H-benzodiazépine-1,3 qui est purifié par recristallisation dans l'éthanol. Rdt : 4,9 g (67 %), F = 206-208°C.

<u>Analyse centésimale</u> $C_{22}H_{22}ClN_3$ M = 363,88

| | C % | H % | Cl % | N % |
|---|---|---|---|---|
| Calculé | 72,61 | 6,10 | 9,74 | 11,55 |
| Trouvé | 72,39 | 6,17 | 9,80 | 11,60 |

En opérant selon les conditions de l'exemple 14 on prépare les produits décrits dans les exemples 15 à 46.

<u>Exemple 15.</u>

<u>(Chloro-4 benzylamino)-2 phényl-5 dihydro-4,5-3H-benzodiazépine-1,3, chlorhydrate</u>

Obtenu par condensation de la chloro-4 benzylamine et de l'iodhydrate de méthylthio-2 phényl-5 dihydro-4,5-3H-benzodiazépine-1,3. F = 181-183°C (acétone-éthanol).

<u>Analyse centésimale</u> $C_{22}H_{21}Cl_2N_3$ M = 398,33.

| | C % | H % | Cl % | N % |
|---|---|---|---|---|
| Calculé | 66,34 | 5,31 | 17,80 | 10,55 |
| Trouvé | 66,30 | 5,38 | 17,78 | 10,55 |

<u>Exemple 16.</u>

<u>Benzylamino-2 chloro-7 phényl-5 dihydro-4,5-3H-benzodiazépine-1,3, chlorhydrate</u>

Obtenu par condensation de la benzylamine et de l'iodhydrate de chloro-7 méthylthio-2 phényl-5 dihydro-4,5-3H-benzodiazépine-1,3. F = 169-170°C (acétone-éthanol).

<u>Analyse centésimale</u> $C_{22}H_{21}Cl_2N_3$ M = 398,33

| | C % | H % | Cl % | N % |
|---|---|---|---|---|
| Calculé | 66,34 | 5,31 | 17,80 | 10,55 |
| Trouvé | 66,30 | 5,48 | 17,67 | 10,49 |

<u>Exemple 17.</u>

<u>Benzylamino-2 (méthyl-4 phényl)-5 dihydro-4,5-3H-benzodiazépine-1,3, chlorhydrate</u>

Obtenu par condensation de la benzylamine et de l'iodhydrate de

(méthyl-4 phényl)-5 méthylthio-2 dihydro-4,5-3H-benzodiazépine-1,3 F = 177-178°C (isopropanol).

Analyse centésimale $C_{23}H_{24}ClN_3$ M = 377,90

| | C % | H % | Cl % | N % |
|---|---|---|---|---|
| Calculé | 73,10 | 6,40 | 9,38 | 11,12 |
| Trouvé | 72,94 | 6,49 | 9,46 | 11,06 |

Exemple 18.

Benzylamino-2 (méthoxy-4 phényl)-5 dihydro-4,5-3H-benzodiazépine-1,3, chlorhydrate

Obtenu par condensation de la benzylamine et de l'iodhydrate de (méthoxy-4 phényl)-5 méthylthio-2 dihydro-4,5-3H-benzodiazépine-1,3. F = 120-122°C (acétone).

Analyse centésimale $C_{23}H_{24}ClN_3O$ M = 393,90

| | C % | H % | Cl % | N % |
|---|---|---|---|---|
| Calculé | 70,13 | 6,14 | 9,00 | 10,67 |
| Trouvé | 70,16 | 6,06 | 9,05 | 10,66 |

Exemple 19.

Furfurylamino-2 phényl-5 dihydro-4,5-3H-benzodiazépine-1,3, hémi-oxalate

Obtenu par condensation de la furfurylamine et de l'iodhydrate de méthylthio-2 phényl-5 dihydro-4,5-3H-benzodiazépine-1,3. F = 220-222°C (éthanol-diméthylformamide).

Analyse centésimale $C_{21}H_{20}N_3O_3$ M = 362,39

| | C % | H % | N % |
|---|---|---|---|
| Calculé | 69,60 | 5,56 | 11,59 |
| Trouvé | 69,30 | 5,99 | 11,44 |

Exemple 20.

Benzylamino-2 (chloro-4 phényl)-5 dihydro-4,5-3H-benzodiazépine-1,3, chlorhydrate

Obtenu par condensation de la benzylamine et de l'iodhydrate de (chloro-4 phényl)-5 méthylthio-2 dihydro-4,5-3H-benzodiazépine-1,3. F = 164-166°C (acétone-éthanol)

Analyse centésimale $C_{22}H_{21}Cl_2N_3$ M = 398,33

| | C % | H % | Cl % | N % |
|---|---|---|---|---|
| Calculé | 66,34 | 5,31 | 17,80 | 10,55 |
| Trouvé | 66,22 | 5,53 | 17,66 | 10,49 |

Exemple 21.

(Chloro-4 benzylamino)-2 (méthyl-4 phényl)-5 dihydro-4,5-3H-benzodiazépine-1,3, chlorhydrate.

Obtenu par condensation de la chloro-4 benzylamine et de l'iodhydrate de (méthyl-4 phényl)-5 méthylthio-2 dihydro-4,5-3H-benzodiazépine -1,3. F = 202-203°C (isopropanol-éther diisopropylique).

Analyse centésimale $C_{23}H_{23}Cl_2N_3$ M = 412,34

| | C % | H % | Cl % | N % |
|---|---|---|---|---|
| Calculé | 67,00 | 5,62 | 17,20 | 10,19 |
| Trouvé | 66,96 | 5,59 | 17,26 | 10,19 |

Exemple 22.

(Méthyl-3 benzylamino)-2 phényl-5 dihydro-4,5-3H-benzodiazépine-1,3, chlorhydrate.

Obtenu par condensation de la méthyl-3 benzylamine et de l'iodhydrate de méthylthio-2 phényl-5 dihydro-4,5-3H-benzodiazépine-1,3. F = 145-146°C (acétone-isopropanol).

Analyse centésimale $C_{23}H_{24}ClN_3$ M = 377,90

| | C % | H % | Cl % | N % |
|---|---|---|---|---|
| Calculé | 73,10 | 6,40 | 9,38 | 11,12 |
| Trouvé | 72,99 | 6,45 | 9,48 | 11,06 |

Exemple 23.

(Méthoxy-4 benzylamino)-2 phényl-5 dihydro-4,5-3H-benzodiazépine-1,3 chlorhydrate

Obtenu par condensation de la méthoxy-4 benzylamine et de l'iodhydrate de méthylthio-2 phényl-5 dihydro-4,5-3H-benzodiazépine-1,3. F = 202-204°C (acétone-éthanol).

Analyse centésimale $C_{23}H_{24}ClN_3O$ M = 393,90

| | C % | H % | Cl % | N % |
|---|---|---|---|---|
| Calculé | 70,13 | 6,14 | 9,00 | 10,67 |
| Trouvé | 70,16 | 6,06 | 9,04 | 10,59 |

Exemple 24.

(Méthyl-4 benzylamino)-2 phényl-5 dihydro-4,5-3H-benzodiazépine-1,3, chlorhydrate

Obtenu par condensation de la méthyl-4 benzylamine et de l'iodhydrate de méthylthio-2 phényl-5 dihydro-4,5-3H-benzodiazépine-1,3, F = 176-178°C (acétone-éthanol).

Analyse centésimale $C_{23}H_{24}ClN_3$ M = 377,90

| | C % | H % | Cl % | N % |
|---|---|---|---|---|
| Calculé | 73,10 | 6,40 | 9,38 | 11,12 |
| Trouvé | 73,16 | 6,10 | 9,50 | 11,30 |

Exemple 25.

Benzylamino-2 méthyl-7 phényl-5 dihydro-4,5-3H-benzodiazépine-1,3,

chlorhydrate

Obtenu par condensation de la benzylamine et de l'iodhydrate de méthyl-7 méthylthio-2 phényl-5 dihydro-4,5-3H-benzodiazépine-1,3. F = 153-155°C (acétone-isopropanol).

Analyse centésimale $C_{23}H_{24}ClN_3$ M = 377,90

| | C % | H % | Cl % | N % |
|---|---|---|---|---|
| Calculé | 73,10 | 6,40 | 9,38 | 11,12 |
| Trouvé | 73,10 | 6,20 | 9,41 | 11,22 |

Exemple 26.

(Méthyl-2 benzylamino)-2 phényl-5 dihydro-4,5-3H-benzodiazépine-1,3, chlorhydrate

Obtenu par condensation de la méthyl-2 benzylamine et de l'iodhydrate de méthylthio-2 phényl-5 dihydro-4,5-3H-benzodiazépine-1,3. F = 131-133°C (acétone-éther diisopropylique).

Analyse centésimale $C_{23}H_{24}ClN_3$ M = 377,90

| | C % | H % | Cl % | N % |
|---|---|---|---|---|
| Calculé | 73,10 | 6,40 | 9,38 | 11,12 |
| Trouvé | 73,10 | 6,16 | 9,39 | 11,26 |

Exemple 27.

Benzylamino-2 (méthyl-3 phényl)-5 dihydro-4,5-3H-benzodiazépine-1,3, chlorhydrate

Obtenu par condensation de la benzylamine et de l'iodhydrate de (méthyl-3 phényl)-5 méthylthio-2 dihydro-4,5-3H-benzodiazépine-1,3. F = 159-161°C (acétone-éthanol).

Analyse centésimale $C_{23}H_{24}ClN_3$ M = 377,90

| | C % | H % | Cl % | N % |
|---|---|---|---|---|
| Calculé | 73,10 | 6,40 | 9,98 | 11,12 |
| Trouvé | 73,05 | 6,45 | 9,40 | 11,06 |

Exemple 28.

Benzylamino-2 (méthyl-2 phényl)-5 dihydro-4,5-3H-benzodiazépine-1,3, chlorhydrate

Obtenu par condensation de la benzylamine et de l'iodhydrate de (méthyl-2 phényl)-5 méthylthio-2 dihydro-4,5-3H-benzodiazépine-1,3. F = 163-164°C (isopropanol-éther diisopropylique)

Analyse centésimale $C_{23}H_{24}ClN_3$ M = 377,90

| | C % | H % | Cl % | N % |
|---|---|---|---|---|
| Calculé | 73,10 | 6,40 | 9,38 | 11,12 |
| Trouvé | 72,90 | 6,60 | 9,40 | 11,08 |

Exemple 29.

(Diméthyl-2,3 benzylamino)-2 phényl-5 dihydro-4,5-3H-benzodiazépine-1,3, chlorhydrate

Obtenu par condensation de la diméthyl-2,3 benzylamine et de l'iodhydrate de méthylthio-2 phényl-5 dihydro-4,5-3H-benzodiazépine-1,3. F = 207-209°C (éthanol).

Analyse centésimale $C_{24}H_{26}ClN_3$ M = 391,92

| | C % | H % | Cl % | N % |
|---|---|---|---|---|
| Calculé | 73,55 | 6,69 | 9,05 | 10,72 |
| Trouvé | 73,60 | 6,69 | 9,06 | 10,69 |

Exemple 30.

(Diméthyl-2,6 benzylamino)-2 phényl-5 dihydro-4,5-3H-benzodiazépine-1,3, chlorhydrate

Obtenu par condensation de la diméthyl-2,6 benzylamine et de l'iodhydrate de méthylthio-2 phényl-5 dihydro-4,5-3H-benzodiazépine-1,3. F = 165-169°C (éthanol).

Analyse centésimale $C_{24}H_{26}ClN_3$ M = 391,92

| | C % | H % | Cl % | N % |
|---|---|---|---|---|
| Calculé | 73,55 | 6,69 | 9,05 | 10,72 |
| Trouvé | 73,44 | 6,88 | 9,04 | 10,69 |

Exemple 31.

Benzylamino-2 chloro-7 (méthyl-4 phényl)-5 dihydro-4,5-3H-benzodiazépine-1,3, chlorhydrate

Obtenu par condensation de la benzylamine et de l'iodhydrate de chloro-7 (méthyl-4 phényl)-5 méthylthio-2 dihydro-4,5-3H-benzodiazépine-1,3. F = 184-186°C (éthanol).

Analyse centésimale $C_{23}H_{23}Cl_2N_3$ M = 412,35

| | C % | H % | Cl % | N % |
|---|---|---|---|---|
| Calculé | 67,00 | 5,62 | 17,20 | 10,19 |
| Trouvé | 67,06 | 5,88 | 16,89 | 10,23 |

Exemple 32.

Benzylamino-2 méthyl-7 (méthyl-4 phényl)-5 dihydro-4,5-3H-benzodiazépine-1,3, chlorhydrate

Obtenu par condensation de la benzylamine et de l'iodhydrate de méthyl-7 (méthyl-4 phényl)-5 méthylthio-2 dihydro-4,5-3H-benzodiazépine-1,3. F = 204-205°C (isopropanol-éther diisopropylique)

Analyse centésimale $C_{24}H_{26}ClN_3$ M = 391,92

| | C % | H % | Cl % | N % |
|---|---|---|---|---|
| Calculé | 73,55 | 6,69 | 9,05 | 10,72 |
| Trouvé | 73,77 | 6,70 | 8,98 | 10,63 |

Exemple 33.

Phényl-5 (pyridyl-2 méthylamino)-2 dihydro-4,5-3H-benzodiazépine-1,3, dichlorhydrate

Obtenu par condensation de l'aminométhyl-2 pyridine et de l'iodhydrate de méthylthio-2 phényl-5 dihydro-4,5-3H-benzodiazépine-1,3. F = 253-255°C (éthanol-diméthylformamide).

Analyse centésimale $C_{21}H_{22}Cl_2N_4$ M = 401,33

| | C % | H % | Cl % | N % |
|---|---|---|---|---|
| Calculé | 62,85 | 5,53 | 17,67 | 13,96 |
| Trouvé | 62,86 | 5,66 | 17,56 | 13,96 |

Exemple 34.

Phényl-5 (pyridyl-3 méthylamino)-2 dihydro-4,5-3H-benzodiazépine-1,3, dichlorhydrate.

Obtenu par condensation de l'aminométhyl-3 pyridine et de l'iodhydrate de méthylthio-2 phényl-5 dihydro-4,5-3H-benzodiazépine-1,3. F = 210-212°C (acétone-éthanol).

Analyse centésimale $C_{21}H_{22}Cl_2N_4$ M = 401,33

| | C % | H % | Cl % | N % |
|---|---|---|---|---|
| Calculé | 62,85 | 5,53 | 17,67 | 13,96 |
| Trouvé | 62,90 | 5,55 | 17,62 | 14,03 |

Exemple 35.

Phényl-5 (pyridyl-4 méthylamino)-2 dihydro-4,5-3H-benzodiazépine-1,3, dichlorhydrate

Obtenu par condensation de l'aminométhyl-4 pyridine et de l'iodhydrate de méthylthio-2 phényl-5 dihydro-4,5-3H-benzodiazépine-1,3. F = 251-253°C (éthanol).

Analyse centésimale $C_{21}H_{22}Cl_2N_4$ M = 401,33

| | C % | H % | Cl % | N % |
|---|---|---|---|---|
| Calculé | 62,85 | 5,53 | 17,67 | 13,96 |
| Trouvé | 62,89 | 5,60 | 17,63 | 13,98 |

Exemple 36.

(Nitro-4 benzylamino)-2 phényl-5 dihydro-4,5-3H-benzodiazépine-1,3, oxalate

Obtenu par condensation de la nitro-4 benzylamine et de l'iodhydrate de méthylthio-2 phényl-5 dihydro-4,5-3H-benzodiazépine-1,3. F = 160-162°C (acétone-éthanol).

Analyse centésimale $C_{26}H_{22}N_4O_6$ M = 462,45

| | C % | H % | N % |
|---|---|---|---|
| Calculé | 62,33 | 4,80 | 12,09 |

| | C % | H % | N % |
|---|---|---|---|
| Trouvé | 62,38 | 4,86 | 12,13 |

Exemple 37.

(Amino-3 chloro-4 benzylamino)-2 phényl-5 dihydro-4,5-3H-benzodiazépine-1,3 maléate

Obtenu par condensation de l'amino-3 chloro-4 benzylamine et de l'iodhydrate de méthylthio-2 phényl-5 dihydro-4,5-3H-benzodiazépine-1,3. F = 166-168°C (acétone-éthanol)

Analyse centésimale $C_{26}H_{25}ClN_4O_4$  M = 492,95

| | C % | H % | Cl % | N % |
|---|---|---|---|---|
| Calculé | 63,35 | 5,11 | 7,19 | 11,37 |
| Trouvé | 63,21 | 5,15 | 7,22 | 11,23 |

Exemple 38.

(Amino-4 benzylamino)-2 phényl-5 dihydro-4,5-3H-benzodiazépine-1,3, dichlorhydrate

Obtenu par condensation de l'amino-4 benzylamine et de l'iodhydrate de méthylthio-2 phényl-5 dihydro-4,5-3H-benzodiazépine-1,3. F = 188-190°C (éthanol-éther).

Exemple 39.

Cyclopentylamino-2 phényl-5 dihydro-4,5-3H-benzodiazépine-1,3 , chlorhydrate

Obtenu par condensation de la cyclopentylamine et de l'iodhydrate de méthylthio-2 phényl-5 dihydro-4,5-3H-benzodiazépine-1,3. F = 181-183°C (acétone-éthanol).

Analyse centésimale $C_{20}H_{24}ClN_3$  M = 341,87

| | C % | H % | Cl % | N % |
|---|---|---|---|---|
| Calculé | 70,26 | 7,07 | 10,37 | 12,29 |
| Trouvé | 70,08 | 7,08 | 10,46 | 12,36 |

Exemple 40.

/(Diméthylamino-3 propyl) amino/-2 phényl-5 dihydro-4,5-3H-benzodiazépine-1,3, dichlorhydrate

Obtenu par condensation de la diméthylamino-3 propylamine et de l'iodhydrate de méthylthio-2 phényl-5 dihydro-4,5-3H-benzodiazépine-1,3. F = 185-187°C (acétone-éthanol).

Analyse centésimale $C_{20}H_{28}Cl_2N_4$  M = 395,36

| | C % | H % | Cl % | N % |
|---|---|---|---|---|
| Calculé | 60,74 | 7,14 | 17,93 | 14,17 |
| Trouvé | 60,48 | 7,47 | 17,86 | 14,15 |

Exemple 41.

Chloro-7 /(diméthylamino-3 propyl)amino/-2 phényl-5 dihydro-4,5-3H-benzodiazépine-1,3, dioxalate

Obtenu par condensation de la diméthylamino-3 propylamine et de l'iodhydrate de chloro-7 méthylthio-2 phényl-5 dihydro-4,5-3H-benzodiazépine-1,3. F = 186-188°C (éthanol).

Analyse centésimale $C_{24}H_{29}ClN_4O_8$ M = 536,96

| | C % | H % | Cl % | N % |
|---|---|---|---|---|
| Calculé | 53,68 | 5,44 | 6,60 | 10,43 |
| Trouvé | 53,26 | 5,66 | 6,61 | 10,37 |

Exemple 42.

/(Diméthylamino-2 éthyl)amino/-2 phényl-5 dihydro-4,5-3H-benzodiazépine-1,3, dichlorhydrate

Obtenu par condensation de la diméthylamino-2 éthylamine et de l'iodhydrate de méthylthio-2 phényl-5 dihydro-4,5-3H-benzodiazépine-1,3. F = 223-225°C (acétone-éthanol).

Analyse centésimale $C_{19}H_{26}Cl_2N_4$ M = 381,34

| | C % | H % | Cl % | N % |
|---|---|---|---|---|
| Calculé | 59,84 | 6,87 | 18,60 | 14,70 |
| Trouvé | 59,50 | 7,35 | 18,48 | 14,70 |

Exemple 43.

Phénéthylamino-2 phényl-5 dihydro-4,5-3H-benzodiazépine-1,3, chlorhydrate

Obtenu par condensation de la phényl-2 éthylamine et de l'iodhydrate de méthylthio-2 phényl-5 dihydro-4,5-3H-benzodiazépine-1,3. F = 164-166°C (acétone-éthanol).

Analyse centésimale $C_{23}H_{24}ClN_3$ M = 377,90

| | C % | H % | Cl % | N % |
|---|---|---|---|---|
| Calculé | 73,10 | 6,40 | 9,38 | 11,12 |
| Trouvé | 73,06 | 6,44 | 9,40 | 11,06 |

Exemple 44.

(Méthyl-4 pipérazinyl-1)-2 phényl-5 dihydro-4,5-3H-benzodiazépine-1,3, dichlorhydrate, hydrate

Obtenu par condensation de la N-méthylpipérazine et de l'iodhydrate de méthylthio-2 phényl-5 dihydro-4,5-3H-benzodiazépine-1,3. F = 193-194°C (éthanol-éther diisopropylique).

Analyse centésimale $C_{20}H_{27}Cl_2N_4O$ M = 410,36

| | C % | H % | Cl % | N % | O % |
|---|---|---|---|---|---|
| Calculé | 58,53 | 6,63 | 17,28 | 13,65 | 3,90 |
| Trouvé | 58,23 | 6,84 | 17,23 | 13,52 | 4,04 |

Exemple 45.

/(Ethyl-1 pyrrolidinyl-2) méthylamino/-2 phényl-5 dihydro-4,5-3H-benzodiazépine-1,3, dichlorhydrate

Obtenu par condensation de l'aminométhyl-2 éthyl-1 pyrrolidine et de l'iodhydrate de méthylthio-2 phényl-5 dihydro-4,5-3H-benzodiazépine-1,3. F = 242-245°C (éthanol).

Analyse centésimale $C_{22}H_{30}Cl_2N_4$ M = 421,40

| | C % | H % | Cl % | N % |
|---|---|---|---|---|
| Calculé | 62,70 | 7,18 | 16,83 | 13,30 |
| Trouvé | 62,85 | 7,13 | 16,83 | 13,26 |

Exemple 46.

/(Hydroxy-2 phényl-2 éthyl)-amino/-2 phényl-5 dihydro-4,5-3H-benzodiazépine-1,3

Obtenu par condensation de l'amino-2 phényl-1 éthanol et de l'iodhydrate de méthylthio-2 phényl-5 dihydro-4,5-3H-benzodiazépine-1,3. F = 174-176°C (éthanol).

Analyse centésimale $C_{23}H_{23}N_3O$ M = 357,43

| | C % | H % | N % |
|---|---|---|---|
| Calculé | 77,28 | 6,48 | 11,76 |
| Trouvé | 77,36 | 6,43 | 11,86 |

Exemple 47.

(Diméthyl-2,2 hydrazino)-2 phényl-5 dihydro-4,5-3H-benzodiazépine-1,3, chlorhydrate

Un mélange de 25 g (63 m.moles) d'iodhydrate de méthylthio-2 phényl-5 dihydro-4,5-3H-benzodiazépine-1,3, de 56,8 g (950 m.moles) de diméthyl-1,1 hydrazine et de 300 cm3 de N,N-diméthylformamide est porté 40 h à 60°C. Le milieu réactionnel est concentré sous pression réduite. Le résidu est repris par de l'eau, alcalinisé fortement par de la potasse en pastilles et extrait au chlorure de méthylène. La phase organique est lavée à l'eau et séchée sur sulfate de sodium. Après élimination du solvant on recueille la (diméthyl-2,2 hydrazino)-2 phényl-5 dihydro-4,5-3H-benzodiazépine-1,3 que l'on transforme en son chlorhydrate selon la technique habituelle. F = 160-162°C (éthanol-acétate d'éthyle).

Analyse centésimale $C_{17}H_{21}ClN_4$ M = 316,83

| | C % | H % | Cl % | N % |
|---|---|---|---|---|
| Calculé | 64,45 | 6,68 | 11,19 | 17,69 |
| Trouvé | 64,55 | 6,60 | 11,19 | 17,60 |

Exemple 48.

/(Phényl-5 dihydro-4,5-3H-benzodiazépine-1,3)yl-2/ carbamate d'éthyle

A une suspension de 8,3 g (35 m.moles) d'amino-2 phényl-5 dihydro-4,5-3H-benzodiazépine-1,3 dans un mélange de 50 cm3 d'eau et de 50 g de glace pilée on ajoute goutte à goutte 1,95 g (18 m.moles) de chloroformiate d'éthyle puis un mélange de 50 cm3 d'eau et de 50 g de glace pilée. On ajoute alors simultanément 1,95 g (18 m. moles) de chloroformiate d'éthyle et une solution de 1,5 g (37,5 m.moles) de soude dans 10 cm3 d'eau. On laisse revenir à température ambiante et agite encore 1 heure. Le précipité est filtré, lavé à l'eau et séché. Il est purifié par recristallisation dans l'éthanol. F = 209-211°C. Rdt : 4,9 g (39 %). I.R : $\bar{\nu}$ (C = O) = 1680 $cm^{-1}$.

Analyse centésimale $C_{18}H_{19}N_3O_2$ M = 309,35.

| | C % | H % | N % |
|---|---|---|---|
| Calculé | 69,88 | 6,19 | 13,58 |
| Trouvé | 69,98 | 5,96 | 13,54 |

Exemple 49.

/(Phényl-5 dihydro-4,5-3H-benzodiazépine-1,3)yl-2/cyanamide

Une solution de 25,4 g (120 m.moles) d'(amino-2 phényl)-2 phényl-2 éthylamine dans 120 cm3 d'éthanol est ajoutée goutte à goutte à une solution de 17,5 g (120 m. moles) de cyanodithioimidocarbonate de diméthyle dans 100 cm3 d'éthanol. On continue l'agitation 1 h à température ambiante et laisse une nuit au repos. Le précipité est filtré, lavé avec un peu d'éthanol et séché. Il est mis en solution dans 200 cm3 de N,N-diméthylformamide. La solution est portée à reflux pendant 30 heures puis évaporée à sec sous pression réduite. Le résidu est purifié par recristallisation dans un mélange méthanol-N,N-diméthylformamide. La /(phényl-5 dihydro-4,5-3H-benzodiazépine-1,3)yl-2/ cyanamide fond à F = 261-263°C. Rdt : 14,8 g (47 %).

I.R. : $\bar{\nu}$ (C ≡ N) = 2160 $cm^{-1}$.

Analyse centésimale $C_{16}H_{14}N_4$ M = 262,30

| | C % | H % | N % |
|---|---|---|---|
| Calculé | 73,26 | 5,38 | 21,36 |
| Trouvé | 73,32 | 5,25 | 21,44 |

REVENDICATIONS

1. Amino-2 phényl-5 benzodiazépines-1,3 caractérisées par la formule I

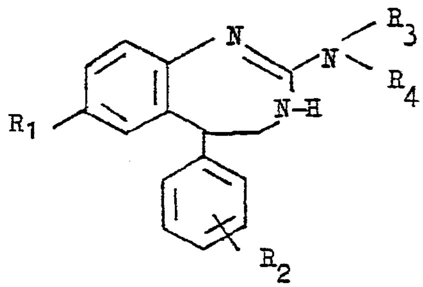

I

dans laquelle $R_1$ et $R_2$ sont l'hydrogène, un atome d'halogène, un radical alcoyle ou alcoxy, $R_2$ pouvant occuper l'une quelconque des positions possibles sur le noyau aromatique ; $R_3$ est l'hydrogène ou un radical alcoyle et alors $R_4$ est l'hydrogène, un radical alcoyle, cycloalcoyle, arylalcoyle , hétéroarylalcoyle, dialcoylaminoéthyle, dialcoylaminopropyle, (éthyl-1 pyrrolidino-2) méthyle, dialcoylamino, alcoxycarbonyle ou cyano, ou bien $R_3$ et $R_4$ forment avec l'atome d'azote adjacent un hétérocyle ayant de 5 à 7 chaînons, contenant éventuellement un autre hétéroatome choisi parmi l'oxygène, le soufre ou l'azote, ce dernier pouvant être substitué par un groupe alcoyle ; et leurs sels d'acides minéraux ou organiques pharmaceutiquement acceptables. Lorsque $R_4$ est le groupe cyano, $R_3$ ne peut être que l'hydrogène.

2. Amino-2 phényl-5 benzodiazépines-1,3 selon la revendication 1 caractérisées en ce que $R_4$ est un groupe alcoxycarbonyle.

3. Amino-2 phényl-5 benzodiazépines-1,3 selon la revendication 1 caractérisées en ce que $R_3$ et $R_4$ sont l'hydrogène.

4. Procédé de préparation des composés selon la revendication 1 à l'exclusion de ceux pour lesquels $R_4$ représente un groupe alcoxycarbonyle ou cyano caractérisé en ce que l'on condense une benzodiazépine de formule

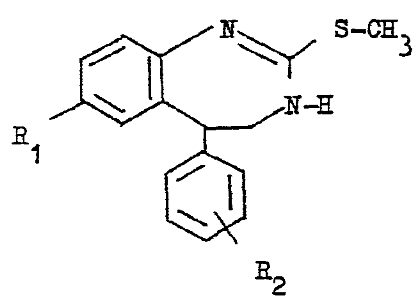

, HI

avec une amine $R_3$-NH-$R_4$, les substituants $R_1$, $R_2$, $R_3$, $R_4$ ayant les significations données précédemment.

5. Procédé de préparation des composés selon la revendication

1, dans la formule I desquels $R_4$ est exclusivement le groupe alcoxy-carbonyle caractérisé en ce que l'on traite une amino-2 benzodiazépine-1,3 de formule

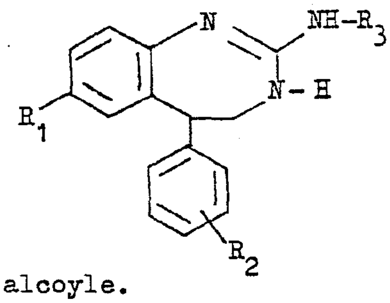

par un chloroformiate d'alcoyle.

6. Procédé de préparation des composés selon la revendication 1 dans la formule I desquels $R_4$ est exclusivement le groupe cyano et $R_3$ est l'hydrogène caractérisé en ce que l'on condense une diamine de formule

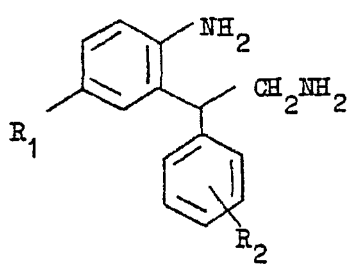

avec le dérivé de formule 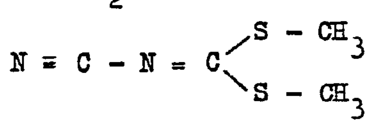

et cyclisation de l'intermédiaire obtenu par chauffage dans un solvant de haut point d'ébullition.

7. Médicaments contenant comme principe actif une amino-2 phényl-5 dihydro-4,5-3H-benzodiazépine-1,3 caractérisée par la formule

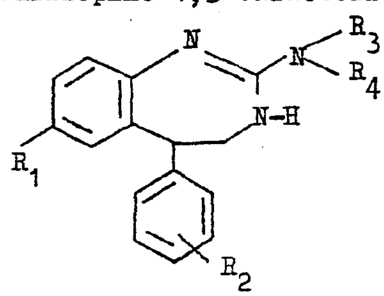

dans laquelle $R_1$ et $R_2$ sont l'hydrogène, un atome d'halogène, un radical alcoyle ou alcoxy, $R_2$ pouvant occuper l'une quelconque des positions possibles sur le noyau aromatique ; $R_3$ est l'hydrogène ou un radical alcoyle et alors $R_4$ est l'hydrogène, un radical alcoyle, cycloalcoyle, arylalcoyle, hétéroarylacoyle, dialcoylaminoéthyle, dialcoylaminopropyle, (éthyl-1 pyrrolidino-2) méthyle, dialcoylamino, alcoxycarbonyle ou cyano, ou bien $R_3$ et $R_4$ forment avec l'atome d'azote

adjacent un hétérocycle ayant de 5 à 7 chaînons, contenant éventuellement un autre hétéroatome choisi parmi l'oxygène, le soufre ou l'azote, ce dernier pouvant être substitué par un groupe alcoyle ; et leurs sels d'acides minéraux ou organiques pharmaceutiquement acceptables.

0117794

Office européen des brevets

# RAPPORT DE RECHERCHE EUROPEENNE

Numéro de la demande

EP 84 40 0233

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int. Cl. 3) |
| D,X<br>Y | US-A-3 681 340 (H.R. RODRIGUEZ)<br>* En entier et en particulier colonne 3, ligne 6 *<br>--- | 1-7 | C 07 D 243/04<br>C 07 D 405/12<br>C 07 D 401/12<br>C 07 D 403/12<br>A 61 K 31/55 |
| Y | US-A-3 838 122 (J.T. SUH)<br>* En entier *<br>--- | 1-7 | |
| D,Y | US-A-3 780 023 (J.T. SUH)<br>* En entier *<br>--- | 1-7 | |
| D,Y | US-A-3 780 024 (J.T. SUH)<br>* En entier *<br>--- | 1-7 | |
| Y | THE MERCK INDEX, 9e dition, 1976, ed. M. Windholz, pub. Merck & Co., Inc., Rahway, USA<br>* Page 394, no. 2961, "Diazepam" *<br>----- | 1-7 | DOMAINES TECHNIQUES RECHERCHES (Int. Cl. 3)<br>C 07 D 243/00<br>C 07 D 405/00<br>C 07 D 401/00<br>C 07 D 403/00 |

Le présent rapport de recherche a été établi pour toutes les revendications

| Lieu de la recherche | Date d'achèvement de la recherche | Examinateur |
|---|---|---|
| LA HAYE | 09-05-1984 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO Form 1503 03.82